# EUROPÄISCHE PATENTSCHRIFT

(11) **EP 2 751 307 B1**
(45) Veröffentlichungstag und Bekanntmachung des Hinweises auf die Patenterteilung: **09.09.2015**
(21) Anmeldenummer: 12726588.2
(22) Anmeldetag: 08.06.2012
(51) Int. Cl.: C25B 1/04, B01D 53/14, C07C 1/12, C07C 29/151, C07C 29/149, C07C 53/02, C10L 3/08, H01M 8/06, C10G 2/00, H01M 8/10

(54) **ENERGIEVERSORGUNGSANLAGE, INSBESONDERE FÜR DEN BEREICH DER HAUSTECHNIK**
POWER SUPPLY SYSTEM, IN PARTICULAR FOR THE FIELD OF BUILDING TECHNOLOGY
INSTALLATION D'ALIMENTATION EN ÉNERGIE, DESTINÉE NOTAMMENT AU DOMAINE DES TECHNOLOGIES DOMESTIQUES

(30) Priorität: 29.08.2011 DE 102011111383
(43) Veröffentlichungstag der Anmeldung: 09.07.2014
(73) Patentinhaber: Busse, Karl-Hermann, 24105 Kiel (DE)
(72) Erfinder: Busse, Karl-Hermann, 24105 Kiel (DE)
(74) Vertreter: Lobemeier, Martin Landolf
(86) Internationale Anmeldenummer: PCT/EP2012/002429
(87) Internationale Veröffentlichungsnummer: WO 2013/029701

(56) Entgegenhaltungen:
- WO-A1-2009/065577
- WO-A1-2010/115983
- WO-A1-2011/061764
- DE-A1-102004 030 717
- DE-A1-102007 019 027
- DE-A1-102009 007 567
- US-A- 4 776 171
- US-A1- 2007 254 969

## Beschreibung

Die Erfindung betrifft eine autarke, dezentrale Energieversorgungsanlage für den Bereich der Haustechnik.

Anlagen mit einer Elektroenergieversorgung, mindestens einem Elektrolyseur zum Erzeugen von Wasserstoff und Sauerstoff, mindestens einem chemischen Reaktor zum katalytischen Umsetzen von Kohlendioxid und des durch Elektrolyse erzeugten Wasserstoffs in eine Langzeit-speicherbare chemische Verbindung hoher Energiedichte, mindestens einen Tank zum Lagern der chemischen Verbindung, sowie mit mindestens einem Wandler zur Stromerzeugung aus der chemischen Verbindung, sowie ein Verfahren zum Betreiben einer entsprechenden Anlage sind als zentrale Großanlagen bekannt, die jedoch den Nachteil eines hohen CO₂ Ausstoßes und eines geringen Wirkungsgrades aufweisen.

Die WO 2009/065577 A1 offenbart eine netzungebundene, d.h. eine ohne Anbindung an einen Verbraucher konzipierte Anlage zur Erzeugung von Kohlenwasserstoffen, bei der aus regenerativer Energie elektrochemisch oder solarthermisch Wasserstoff erzeugt wird, welcher mit aus der Atmosphäre gewonnenem CO₂, zu Kohlenwasserstoffen synthetisiert und anschließend gespeichert wird. Eine autarke, dezentrale Energieversorgungsanlage hohen Wirkungsgrades für den Bereich der Haustechnik ist hier nicht angeregt. Die produzierten Kohlenwasserstoffe müssen vielmehr aufwendig zum jeweiligen Verbraucher transportiert werden.

Auch die WO 2011/061764 A1 offenbart eine großtechnische Herstellung und Speicherung von Kraftstoffen, die durch unregelmäßig auftretende erneuerbare Energien hergestellt werden, wobei zudem nachwachsende Rohstoffe verwendet werden. Auch hier müssen am Ende die Kraftstoffe zum Endverbraucher gebracht werden. Eine komplexe Lösung für eine autarke Energieversorgungsanlage ist nicht angeregt.

Die US 4,776,171 offenbart ebenfalls die großtechnische Ausbeutung erneuerbarer Energien. Hier wird sowohl Windenergie als auch Sonnenenergie verwendet, um Strom zu erzeugen, welcher in Batterien gespeichert werden kann. Weiterhin wird Wasserdampf erzeugt, um Generatoren zu betreiben. In Meerwasserentsalzungsanlagen wird Wasser gewonnen, aus welchem unter Zuhilfenahme des Stroms, Wasserstoff generiert wird. Im Anschluss an eine CO₂-Gewinnungsanlagen wird aus dem Wasserstoff und dem CO₂ Methanol erzeugt, welches später wieder in Verbrennungsmotoren zu elektrischem Strom verarbeitet werden kann. Auch hier wird keine autarke Energieversorgungsanlage hohen Wirkungsgrades zum Einsatz in der Haustechnik angeregt.

Auch die DE 10 2007 019 027 A1 offenbart lediglich die Schritte aus Windenergie elektrische Energie zu erzeugen, welche anschließend zur Elektrolyse von Wasser verwendet wird, wobei aus dem aus der Elektrolyse entstehenden Wasserstoff und Kohlenstoff in einer Synthese Ammoniak oder Methanol erzeugt wird. Auch hier wird lediglich aus regenerativer Energie Elektroenergie erzeugt, die entweder in Akkus gespeichert wird oder zur Erzeugung von Kohlenwasserstoffen dient, wobei die Kohlenwasserstoffe anschließend wieder zu den Endverbrauchern transportiert werden müssen. Eine kompakte Energieversorgungsanlage für die Haustechnik ist auch hier nicht angeregt.

Der Erfindung liegt die Aufgabe zugrunde, eine hocheffizente, dezentrale und weitestgehend CO₂-neutrale sowie autarke Energieversorgungsanlage für den Bereich der Haustechnik sowie ein Verfahren zum Betreiben einer derartigen Anlage zu schaffen.

Zur Lösung der Aufgabe werden die Merkmale des Anspruchs 1 sowie des Anspruch 6 vorgeschlagen. Zweckmäßige Ausgestaltungen der Energieversorgungsanlage ergeben sich aus den Ansprüchen 2 bis 5. Verfahrensmäßig wird die Aufgabe mit Hilfe der Merkmale der Ansprüche 7 bis 14 gelöst.

Die Erfindung bezieht sich somit auf eine hocheffiziente, dezentrale und weitgehend CO₂-neutrale sowie autarke Energieversorgungsanlage, insbesondere für den Bereich der Haustechnik.

Diese besteht vor allem aus einer lediglich der Energieversorgungslange zugeordneten lokalen Elektroenergieversorgung, welcher ein elektrischer Kurzzeitspeicher als Pufferspeicher zugeordnet ist, mit mindestens einem Elektrolyseur zum Erzeugen von Wasserstoff und Sauerstoff, mindestens einem chemischen Reaktor zum katalytischen Umsetzen von Kohlendioxid und des durch Elektrolyse erzeugten Wasserstoffs in Methan, Methanol oder Methansäure als jeweils eine Langzeit-speicherbare chemische Verbindung hoher Energiedichte (Seichermedium), wobei ein Wärmetauscher zur Nutzung der exothermen Prozessenergie bei der Herstellung der chemischen Verbindung vorgesehen ist, mindestens einen Tank zum Zwischenlagern von Kohlendioxid und der chemischen Verbindung, sowie mit mindestens einem Wandler, der aus der chemischen Verbindung Wärme/Kälte und / oder Strom erzeugt, und mit mindestens einem Gasseparator, der aus den Abgasen des mindestens einen Wandlers Kohlendioxid separiert, wobei der zumindest eine Gasseparator über eine Leitung mit dem lokalen Tank verbunden ist, und wobei Wärme- und Stromverbraucher der Haustechnik mit der Energieversorgungsanlage gekoppelt sind.

Die ständige Verteuerung und Verknappung sowie die klimaschädlichen Emissionen von fossilen Energieträgern, wie z.B. Erdöl oder Erdgas, erfordern weitgehend emissionsfreie, dezentrale Energieversorgungsanlagen mit einem hohen Wirkungsgrad, welche die effektive Nutzung von lokal erzeugter Energie, z.B. von erneuerbaren Energieträgern, wie Wind, Wasser und Sonne für den Eigenverbrauch, ermöglichen.

Hierzu ist der Einsatz von Kurzzeit- und Langzeitenergiespeichern zur Zwischenspeicherung nötig.

So muss das zeitliche Ungleichgewicht zwischen der lokalen Erzeugung der erneuerbaren Energie und dem jeweils aktuellen Verbrauch (z.B. bei Tag/Nacht, Sommer/Winter) ausgeglichen werden.

Energiespeicher werden jedoch zum gegenwärtigen Zeitpunkt vornehmlich für den großtechnischen, zentralen Einsatz diskutiert.

Dieser Sachverhalt ist sehr überraschend vergegenwärtigt man sich nachfolgend die in Form einer Aufzählung dargestellten u. a. volkswirtschaftlichen und betriebswirtschaftlichen Vorteile einer dezentralen (lokalen) Energieversorgung und Energiespeicherung:
Die Nutzung erfolgt dezentral. Die Teilnahme der Verbraucher an dem Energiegewinnungsprozess ist inbegriffen.

Die Netzproblematik wird deutlich verringert, da große Strommengen nicht mehr in dem Maße wie bisher transportiert werden müssen. Diese werden überwiegend dort verbraucht, wo diese lokal entstehen.

Der dezentrale chemische Speicher löst das Volumen- und Mengenproblem. Die Speichermenge einer großen Anzahl dezentraler Kleinspeicher ist um ein vielfaches höher, als die weniger zentraler Großspeicher.

Die dezentrale Energieherstellung und -speicherung ist kostengünstiger für den Verbraucher im Vergleich zur zentralen Erzeugung und Speicherung.

Die Möglichkeit der Dauernutzung der alternativen Energien, unabhängig vom jeweiligen Verbrauch, führt zu einer erheblichen Effizienzsteigerung.

Durch die erfindungsgemäße dezentrale chemische Speichertechnologie mit Abgasrückführung wird erstmalig, ortsunabhängig von einer CO₂-Quelle, eine weitgehend emissionsfreie, umweltneutrale Langzeitversorgung (CO₂-neutraler Zyklus an einem Ort) mit Energie bei einem hohen Wirkungsgrad ermöglicht.

Durch einen hohen Gesamtwirkungsgrad (rd. 80%), bei möglicher gleichzeitiger Nutzung von Elektro- und Wärmeenergie, z.B. durch Kraftwärmekopplung (KWK), bei der dezentralen, lokalen, Energieversorgung mittels einem chemischen Energiespeichermedium, wie z.B. synthetisches Erdgas, ergibt sich gegenüber großtechnischen Anwendungen (Gesamtwirkungsgrad bei der Verstromung von synthetischen Erdgas in der Regel maximal rd. 30%) ein beträchtlicher wirtschaftlicher Wettbewerbsvorteil.

Ein weiterer, möglicher Wettbewerbsvorteil liegt in dem Potential der Nutzung von Niedrig- und Negativstrompreisen aus dem Netz. Dadurch ergibt sich eine zusätzlich hohe Wertschöpfung.

Durch die erfindungsgemäße dezentrale, chemische Speichertechnologie ergibt sich zudem die Möglichkeit von energieautarken Insellösungen, die weltweit die Energieversorgung auch in abgelegenen Gebieten der dritten Welt gewährleisten können.

Es besteht sowohl die Möglichkeit stationäre, als auch mobile Lösungen für dezentrale Anwendungen, ortsunabhängig von einer vorhandenen CO₂-Quelle und für verschiedene und große Märkte, wie die Haustechnik umzusetzen.

Der dezentrale Speicher löst das Kostenproblem und Zeitproblem der zentralen Großtechnik. So sind die Entwicklungs- und Investitionskosten des dezentralen Speicherverfahrens im Vergleich zu zentralen großtechnischen Lösungsansätzen erheblich niedriger und gleichzeitig die technische Umsetzung wesentlich schneller und auch mit einem beträchtlich geringeren wirtschaftlichen Risiko verbunden.

Kleinere stoffliche, dezentrale Energiespeicher sind zudem gesellschaftlich leichter umsetzbar, als großtechnische Lösungen.

Eine Zusammenfassung für den großtechnischen, zentralen Einsatz von Energiespeichern gibt "U. Sauer: Optionen zur Speicherung elektrischer Energie in Energieversorgungssystemen mit regenerativer Stromerzeugung, RWTH Aachen, (http://www.eurosolar.de/de/images/stories/pdf/Sauer Optionen Speicher regene rativ okt06.pdf)".

Es bieten sich vornehmlich aus Kostengründen für die Verwendung als Kurzzeitspeicher (meist einige Stunden) für elektrische Energie, insbesondere Batterien auch für die stationäre und mobile, dezentrale Versorgung an. Demgegenüber ist die Energiespeicherung infolge der Nutzung von chemischen Verbindungen als Speichermedien meist wesentlich kostengünstiger für größere Energiemengen und für Langzeitspeicher geeignet.

Eine in den letzten Zeit für großtechnische Anwendungen (vorgesehene elektrische Anschlussleistungen, z.B. für den Elektrolyseur im Megawattbereich) intensiv diskutierte chemische Speicheroption stellt das mit der sog. Sabatier-Reaktion zugängliche Methan dar (auch als SNG bezeichnet - synthetic natural gas). So könnte SNG in das Erdgasnetz eingespeist werden. In das Netz eingebundene, vorhandene Untergrundspeicher könnten ferner in der Form von synthetischem Erdgas insbesondere von Wind- und Solarenergie erzeugte große Energieüberschüsse aufnehmen, vgl. a. WO 2010/115983 A1.

Vorhandene Gaskraftwerke zur variablen Elektroenergieerzeugung sind im Bedarfsfall zum Rückverstromen vorgesehen.

Daher kann im Regelfall die gleichzeitig entstehende Abwärme bei der Verstromung als auch die bei der stark exothermen Methanisierung (Sabatier-Reaktion) entstehende Wärme nicht direkt vor Ort genutzt werden, was zu einem mehr als halbierten Gesamtwirkungsgrad selbst bei der Verwendung sogenannter modernen GuD- Gaskraftwerken und wesentlich höheren Entstehungskosten gegenüber einer dezentralen Anwendung führt, bei der sowohl die Wärme- als auch die Elektroenergie im Sinn einer Kraftwärmekopplung (KWK) (oder auch KraftwärmekälteKopplung KWKK) genutzt werden kann, z.B. wie hier im folgenden beschrieben für den Bereich der Haustechnik. So kann auch im Sinn einer wesentlich schnelleren technischen, kostengünstigen und wirtschaftlich risikoreduzierten Umsetzung für den Bereich der Haustechnik im Vergleich zur Großtechnik auf eine Reihe erprobter und in der Praxis bereits bewährter Komponenten und Sekundäraggregate zur Wärme- und Elektroenergieerzeugung, wie u.a. Blockheizkraftwerken (Mikro-BHKWs), Brennstoffzellen, zurückgegriffen werden.

Die in der WO 2010/115983 A1 beschriebene Technik benötigt zur Versorgung zudem eine stationäre, örtlich vorhandene, leistungsfähige CO- bzw. CO₂-Quelle (Biogasanlage, Verbrennungskraftwerk o. ä.), da sonst infolge der Entnahme von Kohlendioxid aus der Luft, z.B. mittels aufwändiger Aminwäschern der Gesamtwirkungsgrad drastisch auf ein unwirtschaftliches Niveau von « 30% abfällt. Die an eine CO₂-Quelle gebundenen, bekannten Anlagen sind damit nicht ortsunabhängig. Die vorgenannte Verfahrensweise ist zudem über den Gesamtprozess letzt-lich nicht CO₂-neutral, da auch CO₂-emittierende Kraftwerke für die Versorgung mit Kohlendioxid eingesetzt werden und die energetische Nutzung dem technisch zur CO₂- Rückgewinnung nicht ausgestatteten Endverbraucher überlassen wird und so keine umfassende CO₂-Rückführung in den Prozess erfolgen kann.

Demgegenüber ist es bei der erfindungsgemäßen dezentralen Energieversorgungsanlage vorgesehen, den bei der Wärme- und Stromerzeugung anfallenden CO₂-Anteil zum überwiegenden Teil >70% mittels Filterung bzw. Separation zurückzugewinnen. Hierdurch ist die erfindungsgemäße Energieversorgungsanlage nicht örtlich an eine stationäre Kohlendioxidquelle, wie oben beschrieben, gebunden.

Hierzu bietet sich das Verwenden von semipermeablen, ggf. mehrstufigen, Membranfiltern zur Abtrennung von CO₂ und auch von Metalloxiden zur Carbonatbildung (Absorption) mit dem CO₂ und/oder das Abbinden von Kohlendioxid in der Form von Bicarbonaten an. Das Carbonat und/oder Bicarbonat kann später durch einen thermischen und/oder chemischen Aktivierungsschritt benötigtes CO₂ wieder freisetzen.

Nur maximal 30% des benötigten CO₂-Anteiles werden daher dem Reaktionskreislauf wieder von außen neu zugeführt. Dies geschieht vorzugsweise mittels CO₂-Separation aus der Luft, wiederum mit der Hilfe von Membranfiltern, Carbonaten, wie z.B. CaCO₃ und/oder Bicarbonaten, wie z.B. Natriumhydrogencarbonat und anschließenden Aktivierungsschritten, um das CO₂ wieder freizusetzen.

Das nötige CO₂ kann auch z.B. bei landwirtschaftlichen Betrieben, Brauereien zum Sicherstellen der CO₂-Neutralität aus entsprechenden Gärungsprozessen und Biomasse stammen.

Zudem bieten sich auch zusätzliche, separate Reformierungsstufen, z.B. mittels Wasserdampf-Reformierung und/oder der Einsatz von Hochtemperatur-Brennstoffzellen (HT-BZ) mit integrierter Reformierung zur Gewinnung von Kohlendioxid, z.B. aus Erdgas und Alkoholen, wie Methanol, an. Auch durch den optionalen Bezug von Methan aus dem Erdgasnetz und durch Nachtanken des jeweiligen chemischen Speichermediums, vgl. unten, wie Alkohole, z.B. Methanol, Methansäure (Ameisensäure) kann Kohlendioxid in den Prozesskreislauf nachgeführt werden.

Ein weiterer Weg ist die physikalische Separation von CO₂ durch Kühlung und Druck direkt in flüssiger Form aus mittels Wärmetauschern vorgekühlten (sogenannte kryogene Abscheidung von CO₂), auch mit Kohlendioxid angereicherten Abgasen, z.B. von eingesetzten Blockheizkraftwerken.

Da nur ein Anteil von max. 30% CO₂ in den Prozess nachgeführt werden muss, verbleibt der Gesamtwirkungsgrad in einer wirtschaftlich vertretbaren Größenordnung.

Eine vollständige Rückgewinnung des Kohlendioxids aus dem Prozesskreislauf, wie in der DE 10 2008 034 931 A1 beschrieben, ist zwar wünschenswert, aber aufgrund der nicht vollständig zu kontrollierenden Prozessabläufe (z.B. der Verbrennungsreaktionen) gegenwärtig und in absehbarer Zeit aus wirtschaftlichen und technischen Gründen auch für den Bereich der Haustechnik nicht umsetzbar.

"J.Kopyscinski et al., Chem. Eng. Sci., 66 (5) (2011) 924-934", haben in einem Übersichtsartikel den Stand des Wissens der letzten 60 Jahre bzgl. der SNG-Erzeugung unter Nutzung von Kohle und trockener Biomasse zusammengefasst. Den Stand der Technik sowie theoretische Grundlagen und die Arbeiten an der Technischen Universität Graz fasst ebenfalls ein Übersichtsartikel von "T. Kienberger und J. Karl, 11. Symposium Energieinnovation, 10.-12.02.2010, Graz, Austria", zusammen.

Als Sabatier-Reaktion wird die stark exotherme Umsetzung von CO bzw. CO₂ zu Methan und Wasser gemäß der unteren Gleichungen 1 und 2 bezeichnet:
Gl. 1: CO₂ + 4 H₂ → CH₄ + 2 H₂O (ΛH_{R} = -165 kJ/mol (298 K)),
Gl. 2: CO + 3 H₂ → CH₄ + H₂O (ΛH_{R} = -206 kJ/mol (298 K)).

Unter http://people.oregonstate.edu/~atwaterj/h2o gen.htm. sind thermodynamische Daten zugänglich.

Die Reaktion ist fast seit nunmehr 110 Jahren (1902 Sabatier) bekannt und wird mit Hilfe von heterogenen Katalysatoren vorgenommen. Zum Einsatz kommen vor allem Nickel-Katalysatoren, effektiver ist die Verwendung von Ruthenium auf Al₂O₃- oder TiO₂-Trägern.

Problematisch sind die stark exotherme Wärmetönung der Sabatier-Reaktion und die schlechte Wärmeabfuhr bei der Verwendung eines Festbettreaktors, vgl. a.a.O. "T. Kienberger, J. Karl, 11. Symposium Energieinnovation, 10.-12.02.2010, Graz, Austria".

Diese ist ein wesentlicher limitierender Faktor für die Anwendung im Bereich der Haustechnik, da hier ein sehr kompakter Reaktor erforderlich ist und trotzdem eine stabile, reproduzierbare Prozessführung bei wechselndem H₂ mit hohem Wirkungsgrad bei niedrigen Kosten erreicht werden muss.

So ist eine kostenaufwändige und intensive Kühlung beim Festbettreaktor unumgänglich. Außerdem ist die Nutzung der exothermen Abwärme technisch erschwert.

Eine Verdünnung des Eduktgases mit Inertgas ist beispielsweise nicht opportun, da in der Regel die Energieverluste durch die hohe benötigte Gasmenge nicht tragbar wären.

Andererseits sind die Herstellkosten der erfindungsgemäßen Energieversorgungsanlage möglichst niedrig zu halten, um auch dem Privathaushalt die Beschaffung der Technik zu ermöglichen.

Rohrbündelreaktoren sind zudem hinsichtlich einer exothermen Wärmetönung als problematisch einzuschätzen.

Vorteilhaft demgegenüber aus wirtschaftlicher und technischer Sicht für die dezentrale Anwendung im Bereich der Haustechnik ist die Ausführung eines kompakten, ggf. mehrstufigen Reaktors als sogenannter, auch mikrostrukturierter, Wandreaktor.

Das hier zum Einsatz kommende, erfindungsgemäße Konzept unterscheidet sich daher von anderen Anlagenkonzepten auch dadurch, dass ein mikrostrukturierter Reaktor (Gasphase) zur Anwendung kommt, der in der Lage ist, die erhebliche exotherme Reaktionswärme effizient aufzunehmen.

Dabei wird der Katalysator auf entsprechend geformte Bleche aufgebracht, an deren Rückseite ein Kühlmedium (Wärmeträgeröl) zirkuliert.

Weiterer Vorteil der Mikroreaktoren ist es, bei Bedarf die Reaktorleistung durch Parallelisierung der Reaktoren (modulare Bauweise) kostengünstig anzuheben.

Auf Basis dieser Mikroreaktoren ist eine effektive Umsetzung für kleine dezentrale Energieversorgungsanlagen, verbunden mit einer sehr hohen Nutzung der beim

Prozess entstehenden Reaktionswärme, möglich und somit auch ein hoher Gesamtnutzungsgrad der lokal erzeugten regenerativen Energie zu erreichen.

Außerdem soll weitestgehend bei der erfindungsgemäßen Energieversorgungsanlage auf das Verwenden der sehr starken exothermen Umsetzung gemäß Gl. 2, vgl. oben, d.h. CO-haltigen Gasen (z.B. Synthesegas), die in der Regel bei der Großtechnik eingesetzt werden, verzichtet und der Gehalt von Kohlenmonoxid ständig überwacht werden.

So ist Kohlenmonoxid ein starkes Atemgift und in der Haustechnik daher unerwünscht. Außerdem können Konzentrationen von Kohlenmonoxid von rd. >10ppm bereits Brennstoffzellen in ihrer Wirkungsweise stark beeinträchtigen.

In der Regel völlig ausreichend für die dezentrale, lokale Anwendung ist eine elektrische Anschlussleistung, z.B. des vorgesehenen Elektrolyseurs zur Bereitstellung des zum Hydrieren von CO₂ benötigten Wasserstoffs im Bereich von 1 bis 500 kW.

Dieser Leistungsbereich genügt, um Einfamilienhäuser bis hin zu größeren Wohnanlagen, öffentliche Gebäude, wie Krankenhäuser, Bauernhöfe, Brauereien, Hotels sowie kleinere bis mittlere Gewerbebetriebe auch völlig autark ohne Anschluss an eine externe Versorgung, wie dem Strom- bzw. Erdgasnetz, mit chemisch gespeicherter Energie zu versorgen.

Die Speicherkapazität des chemischen Energiespeichers (Tank) ist in der Regel mit rd. 5 bis 35% der jährlich benötigten Energiemenge bemessen, um die hiermit verbundenen Kosten klein zu halten. Dieser Energiespeicher wird daher ggf. mehrfach im Jahr aufgefüllt.

Neben der Haustechnik bietet sich die erfindungsgemäße Energieversorgungsanlage auch für den stationären Einsatz zur Inselversorgung bzw. in infrastrukturell unterversorgten Gebieten (z.B. ohne Elektro-, Gasversorgung und Wasserversorgung) an. Beispielsweise könnte eine erfindungsgemäße Energieversorgungsanlage völlig autark in der Kombination mit Biomasse als H₂- und CO₂-Quelle gemäß Gl. 1, vgl. oben, z.B. auch für die Produktion von Kraft- und Brennstoff in der Form von Methan und/oder Methanol (vgl. a. Gl. 3 unten) und Wasser sorgen.

Außer der Sabatier-Reaktion bieten sich, wie bereits angeführt, noch andere chemische Prozesse und Verbindungen auf Basis der Hydrierung von CO₂, mittels heterogener und/oder homogener Katalysatoren unterstützt, um die nötigen Reaktionen bei moderaten Temperaturen und Drücken vorzunehmen, als chemische Speichermedien (Langzeitspeicher) für die Haustechnik an. Hierzu gehören Alkohole, insbesondere, z.B. Methanol und die Ameisensäure (Methansäure).

So werden zur sogenannten drucklosen Wasserstoffspeicherung gegenwärtig verschiedene organische Verbindungen diskutiert.

Dazu zählen z.B. organische "Hydride" wie Dekalin oder Methylcyclohexan und auch Methanol und Ameisensäure (Methansäure).

So offenbart die DE 10 2009 007 567 A1 eine großtechnische, zentrale Variante der Energiespeicherung mittels dem Speichermedium Methanol.

Zur Herstellung von Methanol wird, wie auch bei der oben angeführten Speicherung mittels Methan (vgl. WO 2010/115983 A1), eine externe und stationäre industrielle Kohlendioxid-Quelle, wie z.B. fossile Kraftwerke, chemische Produktionslinien, zur Versorgung mit Kohlendioxid, im Gegensatz zu der hier dargestellten Erfindung, benötigt.

Ameisensäure bindet rd. 4.4 Gew.-% H₂ und ist zudem sehr einfach zu handhaben. So enthält ein Liter Ameisensäure rund 53 g Wasserstoff, wohingegen das gleiche Volumen von reinem Wasserstoff unter einem Druck von 350 bar nur rund 28 g beträgt. Mit einer Kombination von CO₂-Hydrierung und Ameisensäure-Dehydrierung bietet sich Ameisensäure daher als Hauptprodukt in einem reversiblen Speicherkreislauf an.

Die Nutzung von Ameisensäure als flüssigen Wasserstoffspeicher bietet gegenüber herkömmlichen Speichersystemen signifikante Vorteile. So kann Wasserstoff bereits bei moderaten Drücken und Temperaturen katalytisch (z.B. bei <60 bar, <100°C) mit CO₂ zu Ameisensäure (Methansäure, HCOOH) umgesetzt und später bei milden Bedingungen wieder freigesetzt werden.

Im Gegensatz zu Reformierunsgsprozessen sind dazu keine hohen Temperaturen notwendig. Zudem erfolgt eine selektive Erzeugung von Wasserstoff und Kohlendioxid aus Ameisensäure ohne gleichzeitige Bildung schädlichen Kohlenmonoxids. Damit ist der Wasserstoff direkt in einer Brennstoffzelle mit hohem Wirkungsgrad einsetzbar und die Rückführung des Kohlendioxids zur Hydrolyse mittels einer vergleichsweise einfachen Gasseparation, z.B. mit semipermeablen ggf. mehrstufigen Membranen, technisch machbar. Prinzipiell ist die Erzeugung von Wasserstoff aus Ameisensäure katalytisch bereits ab einer Temperatur von 25 °C möglich.

Mit Hilfe der Ameisensäure als sogenanntes druckloses Speichermedium lassen sich daher die Vorteile der etablierten Wasserstoff-Sauerstoff-Brennstoffzellentechnologie mit denen von flüssigen Kraftstoffen vereinen.

Ameisensäure ist zudem ungiftig. So ist verdünnte Ameisensäure von der US Food and Drug Administration als Nahrungsmittelzusatz zugelassen (US Code of Federal Regulations: 21 CFR 186.1316, 21 CFR 172.515).

Diese ist ferner einfach zu dosieren, zu lagern und mit bereits vorhandener Infrastruktur für flüssige Energieträger kompatibel. Auch deshalb bietet sich die Ameisensäure als chemische Verbindung (Speichermedium) für die Langzeitspeicherung von Elektroenergie an.

Auf der Grundlage einer katalytischen Herstellung von Methan, Methanol, Ameisensäure und der katalytischen Zersetzung von Ameisensäure ist somit die Schaffung einer hocheffizienten, dezentralen und autarken sowie weitgehend CO₂-neutralen, ortsunabhängigen Energieversorgungsanlage, insbesondere für den Bereich der Haustechnik möglich.

Vorteilhafte Ausführungsformen der Erfindung sind in den nachfolgenden drei Zeichnungen dargestellt. Hier zeigen:
- Fig. 1: Eine schematische Darstellung der erfindungsgemäßen Energieversorgunganlage mit den wesentlichen Komponenten und Aggregaten bei der Verwendung von Methan als Speichermedium für die Langzeitspeicherung,
- Fig. 2: Eine schematische Darstellung der erfindungsgemäßen Energieversorgunganlage mit den wesentlichen Komponenten und Aggregaten bei der Verwendung von Methanol als Speichermedium für die Langzeitspeicherung, und
- Fig. 3: Eine schematische Darstellung der erfindungsgemäßen Energieversorgunganlage mit den wesentlichen Komponenten und Aggregaten bei der Verwendung von Methansäure (Ameisensäure) als Speichermedium für die Langzeitspeicherung.

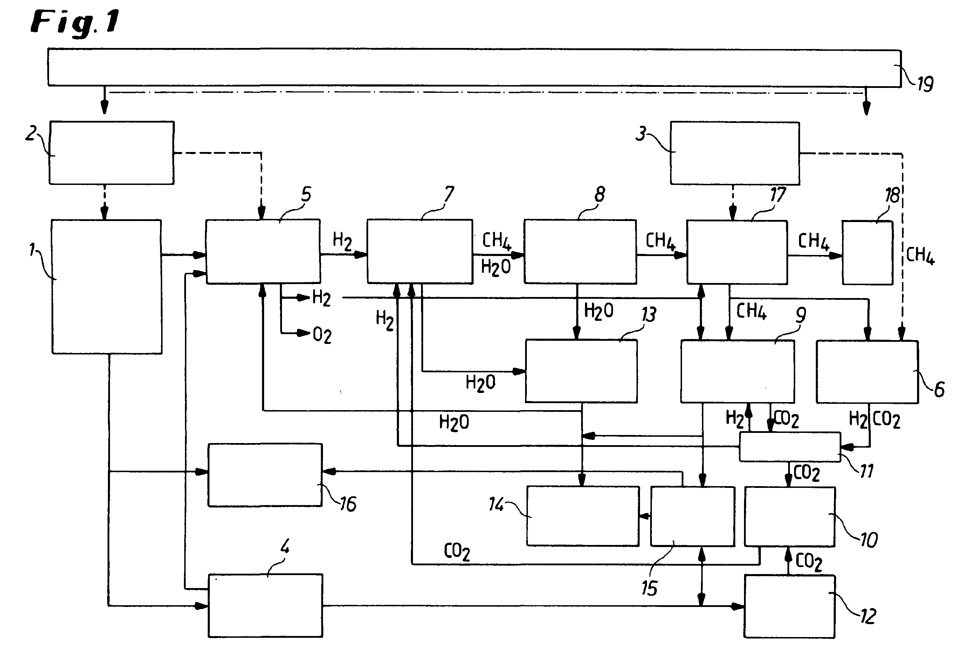

Fig. 1 zeigt schematisch dargestellt eine dezentrale Energieversorgungsanlage, die elektrisch vorzugsweise autark durch eine lokale Elektroenergieversorgung 1, z.B. von Photovoltaik-, Wasserkaft- und Windenergieanlagen versorgt wird. Optional, falls vorhanden, kann die Energieversorgungsanlage durch einen vorhandenen Elektronetzanschluss 2 und/oder einen Erdgasnetzanschluss 3, gespeist werden.

Eine in der Regel zeitlich beschränkte Stromentnahme aus dem Stromnetz 2 bzw. von Erdgas aus dem Gasnetz 3 bietet sich insbesondere an, wenn aufgrund von sehr großen überörtlichen Angebotsmengen, z.B. bei Überproduktion von (regenerativ) erzeugter Energie, der Ankaufspreis sehr günstig ist und diese überschüssige Energie verbraucht und/oder gespeichert werden kann und die lokal meist als Eigenproduktion anfallende Elektroenergie der Elektroenergieversorgung 1 und/oder Methan als Speichermedium nicht oder nur in einem geringen Umfang zur Verfügung steht.

Eine Einspeisung in das überörtliche Strom- und Gasnetz ist durch die erfindungsgemäße dezentrale Energieversorgungsanlage nicht vorgesehen.

Ein zusätzlicher aus Batterien bestehender elektrischer Kurzzeitspeicher 4 als Pufferspeicher stellt bei Bedarf, z.B. zum Anfahren der Systeme, kurzfristig elektrische Energie auch für den Fall von Überlast zur Verfügung. Zudem kann der elektrische Kurzzeitspeicher 4, auch zur Speicherung von überschüssiger Elektroenergie aus der Elektroenergieversorgung 1 im Bereich der Energieversorgungsanlage und zur zusätzlichen Elektroversorgung z.B. des Elektrolyseurs 5, bei zu geringer lokaler Energieerzeugung, zum Beispiel durch Solarzellen, bei Nacht dienen.

Für den Fall das genügend überschüssige, preisgünstige, möglichst regenerativ und lokal erzeugte Elektroenergie 1, 2 zur Verfügung steht, wird hiermit vorzugsweise ein Elektrolyseur 5, zur Aufspaltung von Wasser (H₂O) in Wasserstoff (H₂) und Sauerstoff (O₂) betrieben.

Das für die Elektrolyse nötige Wasser kann durch für die Haustechnik übliche Einspeisungen, z.B. durch öffentliche Wasserleitungen, zur Verfügung gestellt werden und auch, zumindest teilweise, aus der nachfolgenden Methanisierung im Reaktor 7 durch den nachgeschalteten Separator 8 gewonnen werden. Das so erzeugte Wasser kann ferner bei entsprechender Nachbehandlung für andere Zwecke, z.B. für den Trinkwasserbedarf, genutzt werden.

Der erzeugte Wasserstoff (H₂) wird im Fall der Erzeugung von synthetischen Erdgas (Methanisierung) einem oder mehreren ein- oder mehrstufigen kompakten Reaktor(en) 7, zugeführt, in dem katalytisch unterstützt, die stark exotherme, sogenannte Sabatier-Reaktion gemäß Gleichung 1, vgl. oben, abläuft.

Zum Erreichen einer hohen Prozessstabilität und einer homogenen Temperaturverteilung sowie eines hohen Wirkungsgrades ist der Reaktor 7, vorzugsweise zur Methanisierung als ein- oder mehrstufiger auch mikrostrukturierter Wandreaktor ausgelegt. Der mit dem Elektrolyseur 5 erzeugte Wasserstoff (H₂) kann auch direkt oder zusätzlich zum erzeugten Methan in einem Wandler 9 zur Wärme- und/Stromerzeugung, in Brennstoffzellen, in Verbrennungsaggregaten u.a., eingesetzt und gespeichert werden.

Das für die Methanisierungsreaktion im Reaktor 7, nötige Kohlendioxid (CO₂) wird aus einem Kohlendioxidtank 10, zur Verfügung gestellt. Dieser Kohlendioxidtank 10, wird vorzugsweise mit Kohlendioxid, welches bevorzugt aus Verbrennungsabgasen und aus der Luft mittels Membranfiltern gewonnen wird befüllt.

Wie oben bereits ausgeführt trennt nach der stark exothermen Methanisierung im Reaktor 7, die entstandenen Reaktionsprodukte Methan (CH₄) und (heißes) Wasser (H₂O) ein nachgeschalteter Separator 8. Dieses heiße Wasser wird, wie auch das Kühlmittel (vorzugsweise Wasser) des Reaktors 7, zu einem Wärmetauscher 13 geleitet und hierdurch vornehmlich die Warmwasserheizung 14 betrieben.

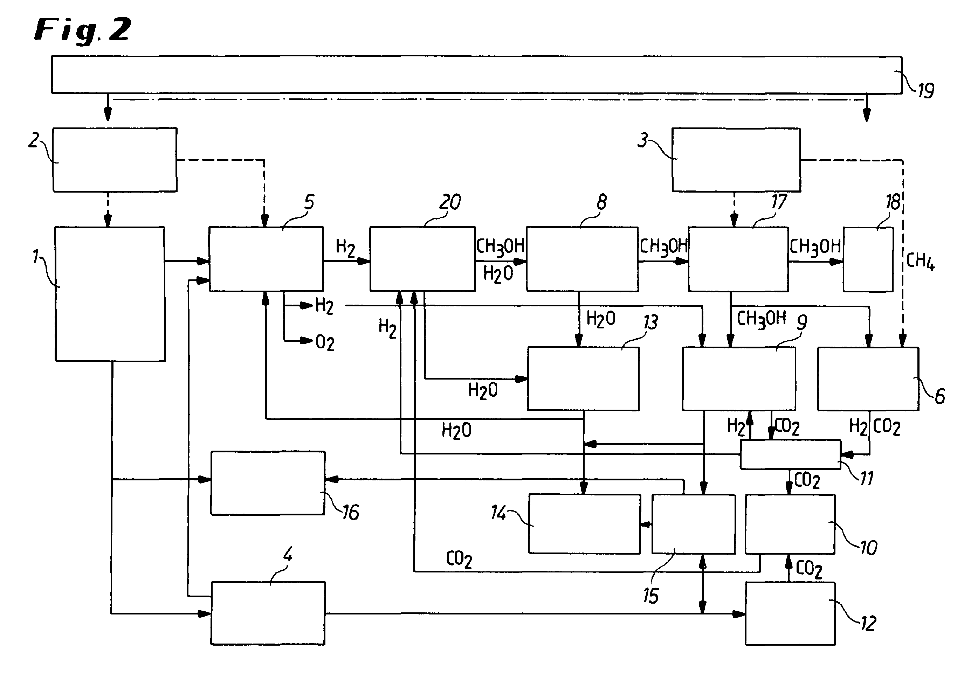

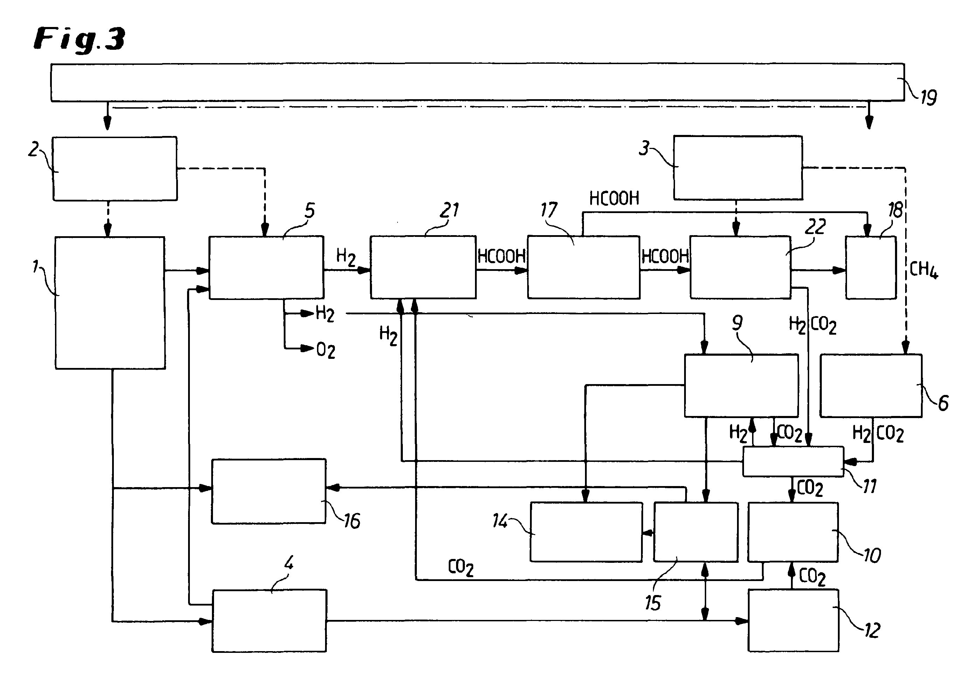

Sofern ausreichend Elektroenergie im Elektroenergieverteiler 15 im Bereich der Energieversorgungsanlage vorhanden ist, kann die Warmwasserheizung 14 auch zusätzlich oder alleine, wie auch andere Aggregate, z.B. eine ggf. in warmen Ländern alternativ vorhandene Klimaanlage 16, vorzugsweise nur durch Elektroenergie betrieben werden. Die im Bereich der Energieversorgungsanlage gemäß Fig.1 (gilt auch für Fig. 2 und Fig. 3) vorhandene Elektroenergie wird zudem für die Vornahme einzelner Regelungen und Steuerungen, u.a. der Prozesssysteme, z.B. mittels Ventilen, Stellmotoren (hier nicht einzeln dargestellt) eingesetzt.

Auch bietet es sich ggf. an Heizungs- und Klimaaggregate im Rahmen z.B einer sogenannten Kraft-Wärme-Kälte-Kopplung direkt mit dem erzeugten Methan (synthetischen Erdgas) zu betreiben (hier nicht zeichnerisch dargestellt).

Das durch den Separator 8 getrennte synthetische Erdgas (Methan, CH₄) wird in einem Tank 17, im gasförmigen oder flüssigen Zustand, gelagert. Dieser Tank 17 erfüllt die Aufgabe eines Zwischenspeichers (Langzeitspeichers) bis zum nächsten abzurufenden Energiebedarf. Je nach Auslegung der Tankgröße kann auch für längere Zeit hiermit die z.B. jahreszeitliche Fluktuation (Winter/Sommer) des lokalen Energieangebots, z.B. durch Photovoltaik, Windenergie und Wasserkraft, ausgeglichen werden. Aus dem Tank 17 und auch über weitere in der Fig.1 nicht dargestellte Zwischentanks, kann überschüssiges, produziertes Energiespeichermedium, hier Methan, auch an externe Verbraucher 18 (z.B. bei dezentral verbundenen Energieversorgungsanlagen) zur weiteren Nutzung abgegeben werden.

Zur Wärme- und Stromerzeugung im Wandler 9, bietet sich ensprechend der jeweils vor Ort erforderlichen Konfiguration, zum Erzielen eines hohen Wirkungsgrades die Kraftwärmekopplung (KWK) bzw. Kraftwärmekältekopplung (KWKK), z.B. der Einsatz sogenannter Blockheizkaftwerke (wie Mikro-BHKWs), und von Gasheizungen, Gasmotoren, Mikro-Turbinen, Nieder- und Hochtemperatur-Brennstoffzellen und sogenannten katalytischen Brennern an.

Ein Steuerungs- und Regelungssystem 19 sorgt letztlich für eine wirtschaftlich optimale Betriebsweise der dezentralen Energieversorgungsanlage abgestimmt auf das jeweilige, zeitabhängige Energieangebot und den Verbrauch (Last).

Durch eine vorhandene Fernabfrage und -steuerung können auch mehrere dezentrale Energieversorgungsanlagen aufeinander eingestellt, abgestimmt und (virtuell) zu einem großen Energiespeicher zusammengeschaltet werden.

Hierdurch ist es beispielsweise möglich beim Angebot von preisgünstigen, z.B. (regenerativen) Strom große Mengen auf dem Markt nachzufragen. Veranschlagt man beispielsweise die mittlere Speicherkapazität einer erfindungsgemäßen Energieversorgungsanlage mit rd. 5 MWh (el) können 1000 haustechnische Installationen bis zu rd. 5 GWh (el) mehrmals im Jahr nachfragen. Dies entspricht etwa der Speichermenge von rd. 5 vergleichsweise großen und teuren Pumpspeicherkraftwerken.

Durch den Vergleich mit Fig. 1 sind verschiedene Betriebsweisen zu erkennen. In Fig. 1 sind zudem nötige Komponenten und Sekundäraggregate dargestellt. Je nach örtlichen Gegebenheiten und Anforderungsprofil müssen diese entsprechend dem Energiebedarf des jeweiligen Betreibers der erfindungsgemäßen Energieversorgungsanlage angepasst werden.

Ein weiteres Ausführungsbeispiel der erfindungsgemäßen Energieversorgungsanlage beim Verwenden von Alkoholen, hier Methanol, als erste chemische Verbindung (Speichermedium) für die Langzeitspeicherung von Energie ist in der folgenden Fig. 2 schematisch dargestellt. Das Ausführungsbeispiel der Energieversorgungsanlage in Fig. 2 unterscheidet sich nur geringfügig zum ersten Ausführungsbeispiel nach in Fig.1.

So wird auch katalytisch unterstützt in der Regel durch einen heterogenen Katalysator, in mindestens einem Reaktor 20, Methanol aus Kohlendioxid und elektrolytisch erzeugten Wasserstoff vorzugsweise gemäß der folgenden Reaktionsgleichung :
Gl.3 : CO₂ + 3H₂ → CH₃OH + H₂O (ΛH_{R} = - 49,6 kJ/mol (298 K)),
mit exothermer Wärmetönung hergestellt.
Ein geeigneter, leistungsfähiger Katalysator wird z.B. in DE 69808983 T2 beschrieben.

Das entstehende Methanol (CH₃OH) wird analog zur Methanisierung, vgl. Fig.1, in einem nachfolgenden Separator 8, vom gleichzeitig entstandenen Wasser getrennt und die Prozess- und Reaktionswärme mittels eines Wärmetauschers 13, zur Warmwasserheizung 14 genutzt.

Anschließend kann das Methanol in einem Tank 17 bis zum Entstehen eines Energiebedarfes zwischengelagert werden. Das Methanol kann dann entweder als Kraft- und Brennstoff zur Wärme- und Stromerzeugung im Wandler 9 genutzt und/oder nach einer Reformierung in der Form von Wasserstoff, z.B. für den Einsatz in Brennstoffzellen bzw. katalytischen Brennern verwendet werden.

Das bei der Reformierung des Methanols entstehende Kohlendioxid kann z.B. mittels semipermeablen Membranen abgetrennt und zur Methanolherstellung dem Reaktor 20 wieder zugeführt werden. Die eintretenden Kohlendioxidverluste können auch analog, wie beim Ausführungsbeispiel Fig. 1 bereits beschrieben, durch Luftseparation, aus Carbonaten, Bicarbonaten und/oder aus der optionalen Erdgasversorgung durch Reformierung des Erdgases ausgeglichen werden.

Auch kann in einfacher Weise durch Nachtanken, z.B. vorzugsweise von CO₂-neutralen, aus Biomasse gewonnenen Methanol und nachfolgendes Reformieren, u.a. mittels Rohr- und Membranreaktoren zur Kohlendioxidseparation, der Kohlendioxidverlust (Kreislaufverlust) ersetzt werden.

Ein weiteres Ausführungsbeispiel der erfindungsgemäßen Energieversorgungsanlage beim Verwenden von Ameisensäure (Methansäure) als erste chemische Verbindung (Speichermedium) für die Langzeitspeicherung von Energie ist in der nachfolgenden Fig. 3 schematisch dargestellt.

Bei diesem Ausführungsbeispiel werden im Vergleich zu den schematischen Darstellungen Fig. 1 und Fig. 2 bevorzugt zwei chemische Reaktoren 21, 22 mit unterschiedlichen Aufgaben verwendet. So dient ein Reaktor 21 zum Herstellen (Hydrieren des Kohlendioxids) von Ameisensäure aus Kohlendioxid und Wasserstoff, wohingegen ein weiterer Reaktor 22 bei Energiebedarf zum Zersetzen (Dehydrieren) der Ameisensäure in Wasserstoff und Kohlendioxid nach erfolgter Zwischenlagerung der Ameisensäure im Tank 17 eingesetzt wird.

Hierbei können beispielsweise im Vergleich zu einem sogenannten Einreaktorkonzept, bei dem der Reaktor 21 und der Reaktor 22 sowie der Tank 17 zu einem einzigen neuen Reaktor mit einem einzigen Katalysator zusammengefasst ist und die Richtung der Reaktion über den Druck und die Temperatur gesteuert wird, die Reaktoren kleiner und effektiver aufeinander hinsichtlich der Umsätze abgestimmt gestaltet werden. Die Lagerung der Ameisensäure kann dann außerhalb der Reaktoren in einem separaten Tank 17 erfolgen.

Ein weiterer wesentlicher Vorteil ist der mögliche vollständig unabhängige Betrieb beider Reaktoren. Zudem können hierbei auch unterschiedliche Katalysatoren eingesetzt werden.

Die Herstellung und Zersetzung der Ameisensäure erfolgt vorzugsweise mit Hilfe von heterogenen und/oder homogenen Katalysatoren, z.B. auf der Basis von Ruthenium, Silber, Palladium und/oder Eisenkomplexen, z.B. Eisen(II)-Komplexen, bei moderaten Drücken und Temperaturen, um einen möglichst hohen Wirkungsgrad zu gewährleisten.

Da das bei der Dehydrierung im Reaktor 22 anfallende Gasgemisch nahezu vollständig aus Wasserstoff und Kohlendioxid besteht, lässt sich dieses vergleichsweise einfach z.B. mittels semipermeablen Membranen separieren und das Kohlendioxid zum Reaktor 21 im Kreislauf für weitere Hydrierungen zurückführen.

Der erhaltene Wasserstoff kann zur Wärme- und Stromerzeugung im Wandler 9 verwendet werden. Hierzu können ähnliche Komponenten und Sekundäraggregate, wie bei den Ausführungsbeispielen Fig. 1 und Fig. 2, z.B. Brennstoffzellen, Verbrennungsaggregate, eingesetzt werden.

Bei einem sehr hohen Energieverbrauch ist es beispielsweise auch möglich die Wasserstofferzeugung durch den Reaktor 22 mit der Wasserstoffproduktion des Elektrolyseurs 5 zu kombinieren. Andere Betriebsweisen können ferner jeweils nach dem zeitlichen Anfall der Elektroenergie und des zeitabhängigen Energieverbrauchs (Last) ausgewählt und eingestellt werden.

An der Stelle des Reaktors 22 ist es zudem möglich auch eine sogenannte Ameisensäure-Brennstoffzelle (in der Fig. 3 nicht dargestellt) in die Energieversorgungsanlage zu integrieren. Auch können noch weitere chemische Reaktoren in die Energieversorgungsanlage integriert werden mit denen die Herstellung von zweiten chemischen Verbindungen, wie z.B. Kraftstoffen und Brennstoffen, möglich ist.

Der optionale Erdgasnetzanschuss 3 kann ferner zur energetischen Versorgung der Energieversorgungsanlage genutzt werden. Dies kann z.B. durch Zumischung von Erdgas zu Wasserstoff oder die alleinige Verwendung von Erdgas zur Wärme-und Stromerzeugung im Wandler 9 geschehen. Beispielweise können die Gasbrenner für die Heizungsanlagen doppelt oder entsprechend für die jeweilige Betriebsweise ausgelegt sein.

Das Erdgas kann auch als Quelle für im Kreislauf verloren gegangenes Kohlendioxid benutzt werden.

So kann Erdgas auch in diesem Ausführungsbeispiel durch einen separaten Reformer 6 und/oder mit einer oder mehreren Hochtemperatur-Brennstoffzellen in CO₂ und Wasserstoff, z.B. mittels eines Nickelkatalysators, aufgetrennt werden.
Der hieraus erzeugte Wasserstoff lässt sich dann einerseits zur weiteren Energieerzeugung verwenden und/oder andererseits gemeinsam mit dem Kohlendioxid für die Produktion der Ameisensäure in Reaktor 21 einsetzen.

Auch kann, wie bei den anderen oben beschriebenen Ausführungsbeispielen, durch einfaches Nachtanken von Ameisensäure der Kohlendioxidverlust ausgeglichen werden.

Im Vergleich zu den anderen Ausführungsbeispielen Fig. 1 und Fig. 2 entfällt bei diesem Beispiel die Nutzung der Reaktionswärme, da diese nur gering ist.

### Bezugszeichenliste

- 1: Elektroenergieversorgung
- 2: Elektronetzanschluss
- 3: Erdgasnetzanschluss
- 4: elektrischer Kurzzeitspeicher
- 5: Elektrolyseur
- 6: Reformer
- 7: Reaktor
- 8: Separator
- 9: Wandler
- 10: Kohlendioxidtank
- 11: Gasseparator
- 12: Gasseparator
- 13: Wärmetauscher
- 14: Warmwasserheizung
- 15: Elektroenergieverteiler
- 16: Klimaanlage
- 17: Tank
- 18: externer Verbraucher
- 19: Steuer- und Regelsystem
- 20: Reaktor
- 21: Reaktor
- 22: Reaktor

## Patentansprüche

1. Autarke, dezentrale Energieversorgungsanlage für den Bereich der Haustechnik, mit einer lediglich der Energieversorgungslange zugeordneten lokalen Elektroenergieversorgung (1), welcher ein elektrischer Kurzzeitspeicher (4) als Pufferspeicher zugeordnet ist, mit mindestens einem Elektrolyseur (5) zum Erzeugen von Wasserstoff und Sauerstoff, mindestens einem chemischen Reaktor (7, 20, 21) zum katalytischen Umsetzen von Kohlendioxid und des durch Elektrolyse erzeugten Wasserstoffs in Methan, Methanol oder Methansäure als jeweils eine Langzeit-speicherbare chemische Verbindung hoher Energiedichte, wobei ein Wärmetauscher (13) zur Nutzung der exothermen Prozessenergie bei der Methanisierung und bei der Herstellung von Methanol vorgesehen ist, mindestens einen Tank (10, 17) zum Zwischenlagern von Kohlendioxid und der chemischen Verbindung, sowie mit mindestens einem Wandler (9), der aus der chemischen Verbindung Wärme und / oder Strom erzeugt, und mit mindestens einem Gasseparator, der aus den Abgasen des mindestens einen Wandlers (9) Kohlendioxid separiert, wobei der zumindest eine Gasseparator über eine Leitung mit dem lokalen Tank (10) verbunden ist, und wobei Wärme- und Stromverbraucher der Haustechnik mit der Energieversorgungsanlage gekoppelt sind.

2. Energieversorgungsanlage nach Anspruch 1,
**dadurch gekennzeichnet,**
**dass** der lokalen Elektroenergieversorgung (1) ein Elektronetzanschluss (2) und/oder dem Tank (17) ein Erdgasnetzanschluss parallel geschaltet ist/sind.

3. Energieversorgungsanlage nach Anspruch 1 oder 2,
**dadurch gekennzeichnet,**
**dass** dem zum Langzeitspeichern erzeugten Methan weniger als 50 % Volumenprozent Wasserstoff zugesetzt ist.

4. Energieversorgungsanlage nach einem der Ansprüche 1 bis 3,
**dadurch gekennzeichnet,**
**dass** der Reaktor (7) zur Methanisierung als ein- oder mehrstufiger, auch mikrostrukturierter, sogenannter Wandreaktor ausgelegt ist.

5. Energieversorgungsanlage nach einem der Ansprüche 1 bis 4,
**dadurch gekennzeichnet,**
**dass** als Wandler (9) zur Wärme- und/oder Stromerzeugung Separatoren (8,11,12) zur Gasabscheidung, Niedertemperatur- und/oder Hochtemperatur-Brennstoffzellen, auch Ameisensäure- Brennstoffzellen, katalytische Brenner, Anlagen zur Heizung mittels Gas und/oder elektrischen Strom, Wärmepumpen, Anlagen zur Stromerzeugung, wie Verbrennungsmotoren, Generatoren, Mikro-Turbinen sowie Anlagen zur Kraftwärmekopplung (BHKWs), Lüftungs- und Klimaanlagen einzeln oder in beliebiger Kombination vorgesehen sind.

6. Verfahren zum Betreiben einer autarken, dezentralen Energieversorgungsanlage für den Bereich der Haustechnik nach einem der Ansprüche 1 bis 5, **dadurch gekennzeichnet,**
**dass** das für die Herstellung von Methan, Methanol oder Methansäure, was jeweils eine Langzeit-speicherbare chemische Verbindung hoher Energiedichte darstellt benötigte Kohlendioxid aus den Verbrennungs- und Zersetzungsprozessen eines Wandlers (9) zur Wärme- und/oder Stromerzeugung separiert, zurückgewonnen und wiederverwendet wird, und der im Kreislauf fehlende Teil des Kohlendioxids durch Separation des Kohlendioxids aus der Luft und/oder aus Verbrennungsprozessen von Methan, Erdgas, Methanol oder Methansäure erzeugt wird.

7. Verfahren nach Anspruch 6,
**dadurch gekennzeichnet,**
**dass** die Separation (11, 12) des CO₂ physikalisch mit Membranfiltern und/oder in der Kombination mit Druckbeaufschlagung und einer Gaskühlung zur Gasabscheidung erfolgt.

8. Verfahren nach Anspruch 6 oder 7,
**dadurch gekennzeichnet,**
**dass** die Wärme- und Stromerzeugung mittels mindestens einer Hochtemperatur-Brennstoffzelle (z. B. SOFC, MCFC) bzw. katalytischen Brennern erfolgt, und das für die chemische Umsetzung des Wasserstoffs nötige CO₂ gleichzeitig mittels Reformierung aus der chemischen Verbindung erzeugt wird.

9. Verfahren nach einem der Ansprüche 6 bis 8,
**dadurch gekennzeichnet,**
**dass** die bei der Wärme- bzw. Stromerzeugung anfallenden Abgase nach einer weiteren Vorreinigung in den Reaktoren (7, 20, 21), gegebenenfalls nach einer Zwischenlagerung, verwendet werden.

10. Verfahren nach einem der Ansprüche 6 bis 9,
**dadurch gekennzeichnet,**
**dass** der durch den Elektrolyseur (5) erzeugte Wasserstoff und Sauerstoff zur Wärme- und/oder Stromerzeugung allein, oder in der Form von Zusätzen zur chemischen Verbindung verwendet wird

11. Verfahren nach einem der Ansprüche 6 bis 10,
**dadurch gekennzeichnet,**
**dass** homogene und/oder heterogene Katalysatoren auf der Basis von Metallen der 6. bis zur 12. Gruppe, insbesondere der 4. bis einschließlich der 6. Periode, des Periodensystems und bevorzugt Ruthenium, Silber, Palladium und Eisen sowie deren Komplexverbindungen zur Hydrierung und Dehydrierung von Ameisensäure dienen.

12. Verfahren nach einem der Ansprüche 6 bis 11,
**dadurch gekennzeichnet,**
**dass** die elektrische Anschlussleistung des Elektrolyseurs (5)von 1 bis 500 kW beträgt.

13. Verfahren nach einem der Ansprüche 6 bis 12,
**dadurch gekennzeichnet,**
**dass** die Speicherkapazität des Tanks (17) insgesamt zwischen 5 bis 35 % des jährlichen Energiebedarfs der Energieversorgungsanlage beträgt.

14. Verfahren nach einem der Ansprüche 6 bis 13,
**dadurch gekennzeichnet,**
**dass** eine Fernabfrage und Fernsteuerung von mindestens einer Energieversorgungsanlage über ein Steuerungs- und Regelungssystem (19) erfolgt, wobei eine Optimierung und Abstimmung von Speicherkapazitäten sowie lokaler und externer Energieerzeugungskapazitäten sowie auch das Zusammenschalten mehrerer Energieversorgungsanlagen zu einem (virtuellen) Großspeicher eine wirtschaftlich verbesserte Nutzung ermöglicht.

## Claims

1. An autonomous, decentralized power supply system for the field of building services, having a local electric power supply (1) only associated with the power supply system, which is assigned an electric short-term storage facility (4) as buffer storage facility, having at least one electrolyser (5) for producing hydrogen and oxygen, at least one chemical reactor (7, 20, 21) for catalytically converting carbon dioxide and the hydrogen, produced by electrolysis, to methane, methanol or methanoic acid as in each case a long-term storable chemical compound of high energy density, a heat exchanger (13) being provided for using the exothermal process energy during methanation and during the production of methanol, at least one tank (10, 17) for the intermediate storage of carbon dioxide and the chemical compound, and at least one converter (8) that uses the chemical compound to generate heat and/or power, and having at least one gas separator that separates carbon dioxide from the exhaust gases of the at least one converter (9), the at least one gas separator being connected to the local tank (10) via a line and heat and power consumers of the building services being coupled to the power supply system.

2. The power supply system according to Claim 1,
**characterized in**
**that** an electricity network confection (2) is connected in parallel to the local electric power supply (1) and/or a natural gas network connection is connected in parallel to the tank (17).

3. The power supply system according to Claim 1 or 2,
**characterized in**
**that** less than 50 viol.-% hydrogen is added to the methane produced for long-term storage.

4. The power supply system according to one of Claims 1 to 3,
**characterized in**
**that** the reactor (7) for methanation is designed a a single or multi stage, also micro-structured, so-called wall reactor.

5. The power supply system according to one of Claims 1 to 4,
**characterized in**
**that** as converter (9) for heat and power generation there are provided separators (8, 11, 12) for gas separation, low-temperature and/or high-temperature fuel cells, also formic-acid fuel cells, catalytic burners, systems for heating by means of gas or electricity, heat pumps, systems for electricity generation, such as internal combustion engines, generators, micro turbines and systems for combined heat and power, ventilation and air-conditioning systems individually or in any Kombination.

6. A method for operating an autonomous, decentralized power supply system for the field of building services according to one of Claims 1 to 5,
**characterized in**
**that** the carbon dioxide from the combustion and decomposition processes of a converter (9) for heat and power generation, required for producing methane, methanol or methanoic acid, being in each case a long-term storable chemical compound of high energy density, is separated, recovered and re-used, and the part of the carbon dioxide missing in the cycle is produced by separation of the carbon dioxide from the air and/or from combustion processes of methane, natural gas, methanol or methanoic acid.

7. The method according to Claim 6,
**characterized in**
**that** the separation (11, 12) of the CO₂ takes places physically using membrane filters and/or in Kombination with pressurization and gas cooling for gas Separation.

8. The method according to Claim 6 or 7,
**characterized in**
**that** the heat and power generation takes place by means of at least one high-temperature fuel cell (e. g. SOFC, MCFC) or catalytic burners and the CO₂ necessary for chemically converting the hydrogen is produced simultaneously from the chemical compound by means of preformation.

9. The method according to one of Claims 6 to 8,
**characterized in**
**that** the exhaust gases produced during heat and power generation are used after further pre-cleaning in the reactors (7, 20, 21), possibly after intermediate storage.

10. The method according to one of Claims 6 to 9,
**characterized in**
**that** the hydrogen and oxygen produced by the electrolyser (5) is used only for heat and/or power generation or in the form of additives to the chemical compound.

11. The method according to one of Claims 6 to 10,
**characterized in**
**that** homogenous or heterogenous catalytic converters based on metals from the 6^{th} to 12^{th} group, in particular the 4^{th} to the 6^{th} period inclusive, of the periodic table and preferably ruthenium, silver, palladium, and iron and their complex compounds serve for hydrogenation and dehydrogenation of formic acid.

12. The method according to one of Claims 6 to 11,
**characterized in**
**that** the installed electric load of the electrolyser (5) is between 1 and 500 kW.

13. The method according to one of Claims 6 to 12,
**characterized in**
**that** the storage capacity of the tank (17) in total is between 5 and 35% of the annual energy demand of the power supply system.

14. The method according to one of Claims 6 to 13,
**characterized in**
**that** remote inquiry and remote control of at least one power supply system takes place by means of open-loop and closed-loop control (19), optimization and tuning of storage capacities and of local and external power generating capacities and also interconnecting several power supply systems to form a (virtual) large storage facility making possible an economically improved use.

## Revendications

1. Dispositif décentralisé d'alimentation en énergie fonctionnant de manière autarcique pour le secteur de la domotique, avec un système d'alimentation énergétique (1) local lui étant exclusivement dédié, à laquelle est rattaché un accumulateur électrique temporaire (4) servant d'accumulateur tampon, avec au moins un électrolyseur (5) permettait l'obtention d'hydrogène et d'oxygène, à au moins un réacteur chimique (7, 20, 21) pour la transformation catalytique du dioxyde de carbone et de l'hydrogène obtenu par électrolyse en méthane, en méthanol ou en acide méthanoïque comme liaison chimique à stockage de longue durée et à haute densité énergétique, à savoir qu'un échangeur thermique (13) est prévu pour l'utilisation de l'énergie de processus exotherme lors de la méthanisation et lors de la fabrication de méthanol, au moins une cuve (10, 17) pour le stockage intermédiaire du dioxyde de carbone et de la liaison chimique, ainsi qu'avec au moins un convertisseur (9) créant de la chaleur et / ou de l'électricité à partir de la liaison chimique, et avec au moins un séparateur de gaz séparant au moins un convertisseur (9) des gaz résiduels, à savoir qu'au moins un séparateur de gaz est relié à la cuve locale (10) via une conduite, et à savoir que les consommateurs domotiques d'énergie thermique et électrique sont reliés au dispositif d'alimentation en énergie.

2. Dispositif d'alimentation en énergie selon la Revendication 1, **caractérisé en ce qu'**un dispositif d'alimentation en gaz naturel est raccordé en parallèle à la cuve, et/ou qu'un dispositif d'alimentation électrique (2) est raccordé en parallèle au dispositif local d'alimentation électrique.

3. Dispositif d'alimentation en énergie selon la Revendication 1 ou 2, **caractérisé en ce que** moins de 50 % du volume d'hydrogène est intégré au méthane obtenu pour un stockage de longue durée.

4. Dispositif d'alimentation en énergie selon une des revendications 1 à 3, **caractérisé en ce que** le réacteur (7) est conçu pour la méthanisation en tant que réacteur mural à une ou plusieurs étapes, et également microstructuré.

5. Dispositif d'alimentation en énergie selon une des revendications 1 à 4, **caractérisé en ce que** des séparateurs (8, 11, 12) sont prévus, seuls ou en combinaison, en tant que convertisseurs (9) pour l'obtention d'énergie thermique et/ou électrique pour la séparation des gaz, les cellules de combustible à basse températere et/ou à haute température, également les cellules de combustible à base d'acide formique, les brûleurs catalytiques, les intallations permettant de chauffer au gaz et/ou à l'électricité, les pompes thermiques, les installations de fabrication électrique comme les moteurs à combustion, les générateurs, les micro-turbines et les installation de cogénérations, les ventilateurs et les climatiseurs.

6. Procédé d'exploitation d'un dispositif d'alimentation énergétique décentralisé pour le secteur domotique selon une des revendications 1 à 5, **caractérisé en ce que** le dioxyde de carbone nécessaire pour l'obtention de méthane, de méthanol ou d'acide méthanoïque, représentant une liaison chimique à stockage longue durée et de grande densité énergétique, issu des processus de combustion et de décomposition d'un convertisseur (9) est séparé pour la création d'énergie thermique et/ou électrique, récupéré et réutilisé, et la partie de dioxyde de carbone manquant dans le circuit est générée par la séparation du dioxyde de carbone de l'air et/ou des processus de combustion du méthane, du gaz naturel, du méthanol ou de l'acide méthanoïque.

7. Procédé selon la revendications 6, **caractérisé en ce que** la séparation (11, 12) du CO₂ se fait physiquement avec des filtres de membrane et/ou en combinaison avec l'application de pression et un refroidissement au gaz, afin d'assurer la réparation des gaz.

8. Procédé selon la revendications 6 ou 7, **caractérisé en ce que** la création d'énergie thermique ou électrique se fait au moyen d'au moins une cellule de combustible à haute température (par ex. SOFC, MCFC) ou des brûleurs catalytiques, et que le CO₂ nécessaire pour la transformation chimique de l'hydrogène est produit simultanément au moyen de la reformation issue de la liaison chimique.

9. Procédé selon une des revendications 6 à 8, **caractérisé en ce que** lors de la fabrication d'énergie thermique / électrique, les gaz résiduels obtenus sont également utilisés dans les réacteurs (7, 20, 21) après un autre nettoyage préalable et, éventuellement, après un stockage intermédiaire.

10. Procédé selon une des revendications 6 à 9, **caractérisé en ce que** l'hydrogène et l'oxygène obtenus par l'électrolyseur (5) est utilisé pour la fabrication d'énergie thermique et/ou électrique, ou, sous forme d'adjuvants, pour la liaison chimique.

11. Procédé selon une des revendications 6 à 10, **caractérisé en ce que** des catalysateurs homogènes et/ou hétérogènes servent, sur la base des métaux du 6ème au 12ème groupe, et en particulier de la 4ème à la 6ème période du système périodique, et de préférence le ruthemium, l'argent, le palladium et le fer, ainsi que leurs liaisons complexes, pour l'hydratation et la déshydratation de l'acide formique.

12. Procédé selon une des revendications 6 à 11, **caractérisé en ce que** le câble de raccordement électrique de l'électrolyseur (5) est de 1 à 500 kW.

13. Procédé selon une des revendications 6 à 12, **caractérisé en ce que** la capacité de stockage de la cuve (17) représente au total 5 à 35 % du besoin énergétique annuel de l'installation d'alimentation énergétique.

14. Procédé selon une des revendications 6 à 13, **caractérisé en ce qu'**une consultation et une commande distance d'au moins une installation d'alimentation énergétique se fait via un système de commande et de régulation (19), à savoir qu'une optimisation et un ajustement des capacités de stockage, ainsi que les capacités locales et externes de génération énergétique, ainsi que l'interconnexion de plusieurs installation d'alimentation énergétique en un collecteur (virtuel) de grande capacité permet d'optimiser l'utilisation.
